(19) Europäisches Patentamt
European Patent Office
Office européen des brevets

(11) EP 0 823 418 B1

(12) **EUROPÄISCHE PATENTSCHRIFT**

(45) Veröffentlichungstag und Bekanntmachung des Hinweises auf die Patenterteilung:
**09.10.2002 Patentblatt 2002/41**

(51) Int Cl.7: **C07C 255/41**, A61K 7/42, G03C 1/815, C07C 309/59

(21) Anmeldenummer: **97112936.6**

(22) Anmeldetag: **28.07.1997**

# (54) Indanylidenverbindungen und ihre Verwendung als UV-Absorber

Indanylidene derivatives and their use as UV-absorbers

Dérivés d'indanylidène et leur utilisation comme absorbateurs d'UV

(84) Benannte Vertragsstaaten:
**CH DE ES FR GB GR IE IT LI NL SE**

(30) Priorität: **07.08.1996 DE 19631863**

(43) Veröffentlichungstag der Anmeldung:
**11.02.1998 Patentblatt 1998/07**

(73) Patentinhaber: **HAARMANN & REIMER GMBH**
**D-37601 Holzminden (DE)**

(72) Erfinder:
- **Koch, Oskar, Dr.**
  **37079 Göttingen (DE)**
- **Surburg, Horst, Dr.**
  **37603 Holzminden (DE)**
- **Langner, Roland**
  **37639 Bevern (DE)**
- **Sommer, Horst, Dr.**
  **37603 Holzminden (DE)**

(74) Vertreter: **Petrovicki, Wolfgang, Dr. et al**
**Bayer AG**
**Konzernbereich RP**
**Patente und Lizenzen**
**51368 Leverkusen (DE)**

(56) Entgegenhaltungen:
**EP-A- 0 670 298** **DE-A- 2 816 819**

- **ARTHUR C. COPE ET AL.: "The Rearrangement of Allyl Groups in Three-Carbon Systems. V. Ethyl (3-Indenyl)-allylcyanoacetate" JOURNAL OF THE AMERICAN CHEMICAL SOCIETY., Bd. 71, Nr. 5, 19.Mai 1949, DC US, Seiten 1589-1593, XP002053862**
- **E. C. HORNING ET AL.: "Dimethoxyindanyl and -indenyl Derivatives" JOURNAL OF THE AMERICAN CHEMICAL SOCIETY, Bd. 76, Nr. 6, 30.März 1954, DC US, Seiten 1700-1702, XP002053863**
- **A. K. BAHL ET AL.: "Nuclear Magnetic Resonance Evidence Regarding the Stereochemistry of Some Cyanoindanylidene Compounds" JOURNAL OF THE CHEMICAL SOCIETY, SECTION C: ORGANIC CHEMISTRY., 1971, LETCHWORTH GB, Seiten 1583-1585, XP002053864**
- **E. CAMPAIGNE ET AL.: "Ring Closure of Ylidenemalonitriles. II. Steric Effects of a Ring at the beta-Position" JOURNAL OF ORGANIC CHEMISTRY, Bd. 28, Nr. 3, 22.März 1963, EASTON US, Seiten 623-624, XP002053865**
- **RICHARD SOMMERVILLE: "Synthesis and Pharmacological Evaluation of Aromatic Dihydroxylated Spiro[indan-1,3'-pyrrolidine] and Spiro[indan-2,2'-pyrrolidine] Derivatives" JOURNAL OF PHARMACEUTICAL SCIENCES., Bd. 74, Nr. 5, Mai 1985, WASHINGTON US, Seiten 553-555, XP002053866**
- **JOSEPH H. CHAN ET AL.: "2,4-Diamino-5-benzylpyrimidines as Antibacterial Agents. 14. 2,3-Dihydro-1-(2,4-diamino-5-pyrimidyl)-1H -indenes as Conformationally Restricted Analogues of Trimethoprim" JOURNAL OF MEDICINAL CHEMISTRY, Bd. 34, Nr. 2, Februar 1991, WASHINGTON US, Seiten 550-555, XP002053867**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **SWATI DAS ET AL.: "Aryl Participated Cyclisations Involving Indane Derivatives A Total Synthesis of (+/-)-Isolongifolene" TETRAHEDRON LETTERS, Bd. 33, Nr. 9, 25.Februar 1992, OXFORD GB, Seiten 1229-1232, XP002053868**
- **N. F. EWEISS ET AL.: "Knoevenagel-Type Condensation of Indan-1-one with Active Methylene Compounds" REVUE ROUMAINE DE CHIMIE, Bd. 24, Nr. 11-12, 1979, BUCAREST, Seiten 1485-1489, XP002053869**
- **BASUDEB BASU ET AL.: "Studies on Intramolecular Cyclisations. Synthesis of Ring Systems Related to Sesquiterpenes" SYNTHETIC COMMUNICATIONS, Bd. 11, Nr. 10, 1981, NEW YORK AND BASEL, Seiten 803-809, XP002053870**
- **SUKANTA BHATTACHARYYA ET AL.: "Selective Reduction of Dienones. Intermediates for Sesqui- and Diterpenes" SYNTHETIC COMMUNICATIONS, Bd. 19, Nr. 3&4, 1989, NEW YOR AND BASEL, Seiten 673-678, XP002053871**
- **M. AADIL ET AL.: "Synthesis of 4,5 Disubstituted 2-Chloronicotinates" SYNTHETIC COMMUNICATIONS, Bd. 23, Nr. 18, 1993, NEW YORK AND BASEL, Seiten 2587-2592, XP002053872**
- **DATABASE WPI Section Ch, Week 9225 Derwent Publications Ltd., London, GB; Class D21, AN 92-204486 XP002053873 & JP 04 134 043 A (KAO CORP) , 7.Mai 1992**


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft neue Indanylidenverbindungen, kosmetische und pharmazeutische Zubereitungen enthaltend Indanylidenverbindungen und die Verwendung als UV-Absorber z.B. in kosmetischen Mitteln, insbesondere in Sonnenschutzmitteln, Tagespflegeprodukten und Haarpflegeprodukten, aber auch zur Verbesserung der Lichtbeständigkeit von techn. Produkten wie Anstrichen, Lacken, Kunststoffe, Textilien, Verpackungsmaterialien und Kautschuken.

UV-Strahlen werden je nach Wellenlänge als UV-A-Strahlen (320-400 nm, UV-A-I: 340-400 nm, W-A-II: 320-340 nm) oder UV-B-Strahlen (280-320 nm) bezeichnet. Ganz allgemein gilt: Die schädigende Wirkung der UV-Strahlen auf die menschliche Haut steigt mit sinkender Wellenlänge und steigender Dauer der Exposition.

So können UV-Strahlen Hautschädigungen hervorrufen, wobei die UV-B-Strahlung einen Sonnenbrand (Erythem) bis hin zu schwersten Hautverbrennungen verursachen kann. Sehr häufige und ungeschützte Bestrahlung der Haut mit Sonnenlicht führt auch zu einem Verlust der Hautelastizität und zu vermehrter Faltenbildung, insgesamt zu frühzeitiger Alterung der Haut. In extremen Fällen können krankhafte Hautveränderungen bis hin zum Hautkrebs auftreten.

Die UV-A-Strahlung bewirkt eine rasche, schwache Direktpigmentierung der Haut. UV-A-Strahlen dringen in tiefere Hautschichten ein und können dort den Alterungsprozeß der Haut beschleunigen. Die kürzerwellige UV-A-II-Strahlung unterstützt die Bildung von Sonnenbrand. Weiterhin kann die UV-A-Strahlung phototoxische oder photoallergische Hautreaktionen auslösen. Es existieren gesicherte Zusammenhänge zwischen UV-A-Exposition und erhöhtem Hautkrebsrisiko.

Entsprechend der Lage ihrer Absorptionsmaxima werden UV-Absorber für kosmetische und pharmakologische Präparate in UV-A- und UV-B-Absorber eingeteilt; wird von einem UV-Absorber sowohl UV-A und UV-B absorbiert, spricht man in diesem Fall von einem UV-A/B-Breitbandabsorber.

Als UV-Absorber sind bereits die verschiedensten Verbindungen, wie Octyltriazon (DE-A 3 206 398), 2-Hydroxy-4-methoxybenzophenon (US 3 751 563) und 4-tert.-Butyl-4'-methoxy-dibenzoylmethan (DE-A 2 945 125) vorgeschlagen worden. Diese Verbindungen haben entweder nicht die gewünschte breite UV-A- und UV-B-Absorption oder besitzen nur eine geringe Absorption in diesem Bereich oder sind nicht genügend photostabil.

Der Erfindung liegt deshalb die Aufgabe zugrunde, verbesserte UV-A- und UV-B-Breitbandabsorber zur Verfügung zu stellen.

Die Erfindung betrifft die Verwendung von Verbindungen der Formel (I)

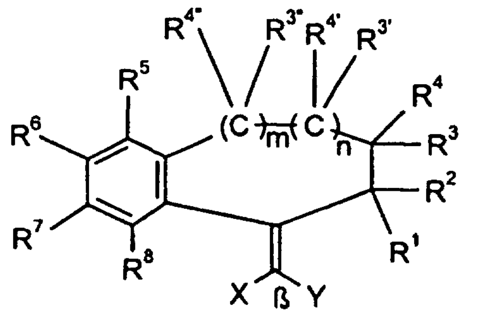

(I)

worin

| | |
|---|---|
| $R^1$ bis $R^4$, $R^{3'}$, $R^{3''}$, $R^{4'}$ und $R^{4''}$ | unabhängig voneinander Wasserstoff, $C_1$-$C_8$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe, insbesondere $C_3$-$C_4$-Alkylen, bedeuten können. wobei eine Methylengruppe durch -O-, -S-, oder -NH- ersetzt sein kann, weiterhin unabhängig voneinander $C_1$-$C_4$-Alkoxy, Hydroxy, Carboxy, Carbalkoxy oder Carbamoyl bedeuten, |
| $R^5$ bis $R^8$ | unabhängig voneinander die Bedeutung von $R^1$, $R^2$ oder Sulfo oder Aminosulfonyl haben, |
| X und Y | unabhängig voneinander CN, $CO_2R^9$, $CONR^9R^{10}$ oder $COR^9$ bedeuten, wobei $R^9$ und $R^{10}$ unabhängig voneinander für Wasserstoff, $C_1$ bis $C_8$-Alkyl oder $C_5$-$C_{10}$-Cy- |

cloalkyl stehen und weiterhin einer der Reste

X oder Y zusätzlich ein $C_1$-$C_8$-Alkylrest, ein $C_5$-$C_{10}$-Arylrest, insbesondere Phenyl, oder ein 5- bis 6-gliedriger Heteroarylrest welcher 1 bis 2 Heteroatome aus der Reihe N, O, S enthält sein kann, oder

X und Y zusammen mit dem β-Atom, an welches sie gebunden sind, einen 5- bis 7-gliedrigen Ring bedeuten, der bis zu 3 Heteroatome, insbesondere Sauerstoff und/oder Stickstoff enthält, wobei die Ringatome substituiert sein können, insbesondere mit exocyclisch doppelt gebundenem Sauerstoff (Ketogruppe), bevorzugt in Nachbarstellung zu dem β-Atom,

und

n, m unabhängig voneinander Null oder 1 bedeuten,

als UV-Absorber, vorzugsweise in Sonnenschutzmitteln.

Ein weiterer Teil der Erfindung betrifft kosmetische und pharmazeutische Zubereitungen enthaltend die Indanyliden-Verbindungen der Formel (I).

Aus der EP-A 670 298 sind Verbindungen der Formel

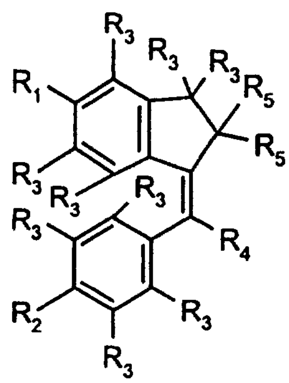

bekannt, worin

$R_1$, $R_2$, und $R_3$ jeweils unabhängig voneinander gleich oder verschieden sein können und Wasserstoffatom, Halogenatom, Hydroxygruppe, Nitrogruppe, Cyanogruppe oder einwertiger, gegebenenfalls substituierter organischer Rest bedeuten und

$R_4$ Wasserstoffatom, Halogenatom, Nitrogruppe, Cyanogruppe oder einwertiger, gegebenenfalls substituierter organischer Rest bedeutet und

$R_5$ gleich oder verschieden sein kann und Halogenatom, Hydroxygruppe, Nitrogruppe, Cyanogruppe oder einwertiger, gegebenenfalls substituierter organischer Rest bedeutet.

Weiterhin sind die nachfolgenden Verbindungen bekannt:

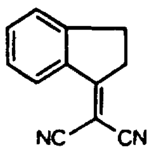
I

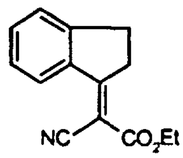
II

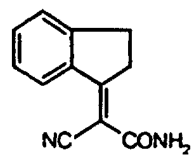
III

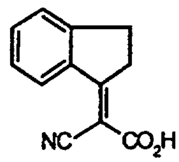
IV

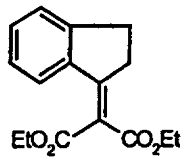
V

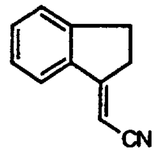
VI

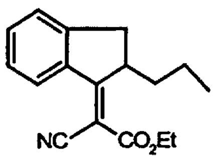
VII

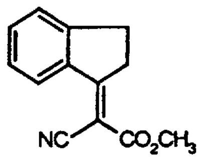
VIII

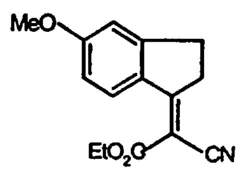
IX

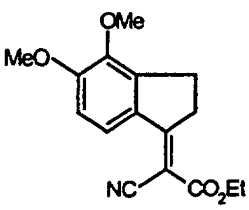
X

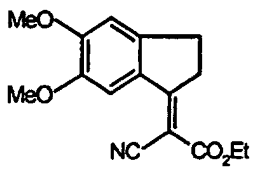
XI

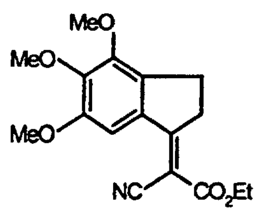
XII

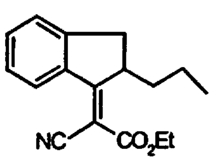
VII

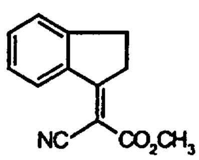
VIII

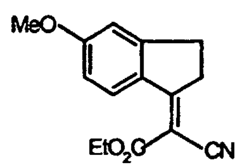
IX

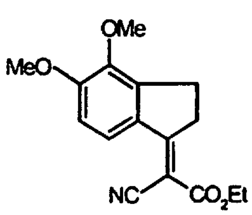
X

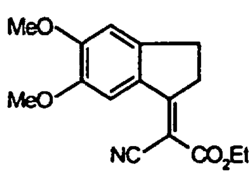
XI

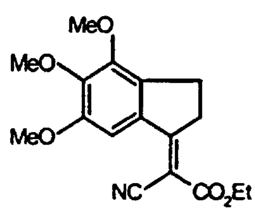
XII

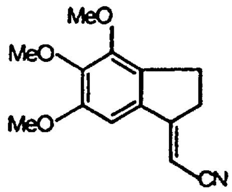

XIII

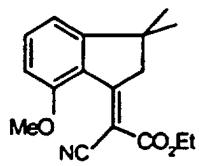

XIV

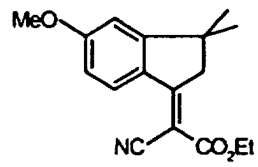

XV

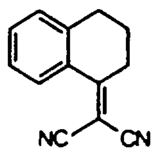

XVI

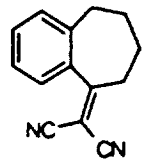

XVII

Die Verbindungen sind bekannt aus:

- JOURNAL OF THE AMERICAN CHEMICAL SOCIETY., Bd. 71, Nr. 5, 19. Mai 1949, DC US, Seiten 1589-1593 (Verbindungen II und VII)
- JOURNAL OF THE AMERICAN CHEMICAL SOCIETY., Bd. 76, Nr. 6, 30. März 1954, DC US, Seiten 1700-1702 (Verbindung X)
- JOURNAL OF THE CHEMICAL SOCIETY., SECTION C: ORGANIC CHEMISTRY., 1971, LETCHWORTH GB, Seiten 1583-1585 (Verbindungen I, II, III, IV, V, VI und VIII)
- JOURNAL OF ORGANIC CHEMISTRY, Bd. 28, Nr. 3, 22. März 1963, EASTON US, Seiten 623-624 (Verbindungen I, XVI und XVII)
- JOURNAL OF PHARMACEUTICAL SCIENCES, Bd. 74, Nr. 5, Mai 1985, WASHINGTON US, Seiten 553-555 (Verbindungen X und XI)
- JOURNAL OF MEDICINAL CHEMISTRY, Bd. 34, Nr. 2, Februar 1991, WASHINGTON US, Seiten 550-555 (Verbindungen XII uns XIII)
- TETRAHEDRON LETTERS, Bd. 33, Nr. 9, 25. Februar 1992, OXFORD GB, Seiten 1229-1232 (Verbindung XIV)
- REVUE ROUMAINE DE CHIMIE, Bd. 24, Nr. 11-12, 1979, BUCAREST, Seiten 1485-1489 (Verbindungen II und IV)
- SYNTHETIC COMMUNICATIONS, Bd. 11, Nr. 10, 1981, NEW YORK AND BASEL, Seiten 803-809 (Verbindung IV)
- SYNTHETIC COMMUNICATIONS, Bd. 19, Nr. 3&4, 1989, NEW YORK AND BASEL, Seiten 673-678 (Verbindung XV)

Weiterer Gegenstand der Erfindung sind deshalb Verbindungen der Formel (I) mit Ausnahme der vorstehend genannten Verbindungen.

Ein bevorzugter Gegenstand der Erfindung sind Verbindungen der Formel (I), worin X und Y keinen unsubstituierten oder substituierten Phenylring bedeuten, sofern

$$m = n = \text{Null}.$$

Die Eigenschaften der Verbindungen der Formel (I) können durch geeignete Wahl der Substituenten innerhalb weiter Grenzen variiert werden. Dies gilt insbesondere sowohl für die Lage des Absorptionsmaximums (so liegt z.B. im Fall von $R^6$ = Alkoxy das Maximum im UV-A-Bereich, bei $R^5$-$R^8$ = H und n, m = 0, $R^1$-$R^4$ = H im UV-B-Bereich, im Fall von $R^7$ = Alkyl werden sogar der UV-A- und der UV-B-Bereich abgedeckt) als auch für die Wasser- und Öllöslichkeit (Sulfonsäuregruppen im aromatischen Ring fördern die Wasserlöslichkeit, bei Abwesenheit von Sulfonsäuregruppen sind die Verbindungen der Formel (I) vornehmlich öllöslich).

Bevorzugte Verbindungen sind solche, in denen X Cyan und Y Carb-$C_1$-$C_4$-alkoxy bedeuten. Desweiteren sind bevorzugte Verbindungen solche, bei denen $R^6$ Alkyl oder besonders Alkoxy bedeutet; sie weisen eine hohe Extinktion auf. Die bevorzugten kosmetischen und pharmazeutischen Zubereitungen enthalten bevorzugt die Verbindungen der Formel (I) entsprechend den Formeln

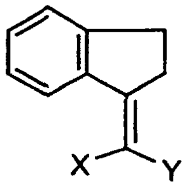

(Ia)

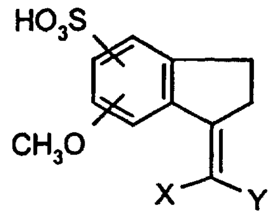

(Ib)

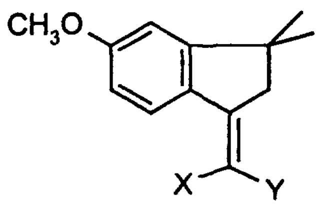

(Ic)

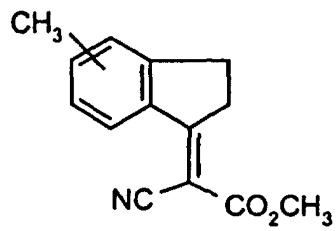

(Id)

Die erfindungsgemäßen Verbindungen eignen sich besonders gut für die Verwendung in Sonnenschutzmitteln, vorzugsweise in kosmetischen und pharmazeutischen Präparaten aber auch als Alterungsschutzmittel für technische Produkte. Sie zeichnen sich durch eine ausgezeichnete Lichtstabilität aus.

Die Verbindungen der Formel (I) können durch (Knoevenagel)-Kondensation von Verbindungen der Formel (II)

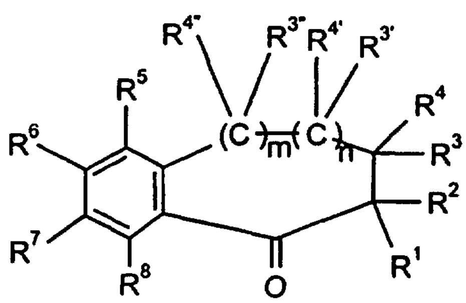

(II)

wobei $R^1$ bis $R^8$ die oben genannte Bedeutung haben,
mit Verbindungen der Formel (III)

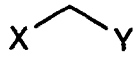

(III)

mit der für X und Y vorgenannten Bedeutung
hergestellt werden (vgl. Organikum, VEB Deutscher Verlag, Berlin 1986, S. 459) und sind mit guten bis sehr guten Ausbeuten zu erhalten.

Die hierfür verwendeten Indanone können durch F.C.-Reaktion von (substituierten) Acrylsäureestern mit (substituierten) Aromaten oder im Fall von Hydroxysubstituenten ($R^5$-$R^8$) durch Fies'sche Umlagerung entsprechender

Phenolester hergestellt werden.

Die erfindungsgemäßen UV-Absorber der Formel (I) besitzen eine glückliche Kombination wünschenswerter Eigenschaften, nämlich

- hohe UV-Schutzleistung bei bereits niedrigen Einsatzkonzentrationen,
- ausgezeichnete Lichtstabilität,
- ausgezeichnete Thermostabilität,
- gute Löslichkeit in kosmetischen Lösungsmitteln und ausgezeichnete Löslichkeit der kristallinen, öllöslichen UV-Absorber in flüssigen, öllöslichen Absorbern wie p-Methoxyzimtsäure-ethyl-, -isoamyl und -isooctylester, Ethylhexylsalicylat, Homomenthylsalicylat, Menthylanthranilat, p-Aminobenzoesäureethylhexylester, 3,3-Diphenyl-2-cyanoacrylsäure-ethyl- und -ethylhexylester oder Kombinationen fllüssiger, öllöslicher UV-Absorber,
- Verträglichkeit mit kosmetischen Grundstoffen,
- pH-Stabilität,
- problemlose Verarbeitbarkeit in kosmetischen Formulierungen und Stabilität unter Anwendungsbedingungen,
- Verträglichkeit mit Verpackungsmaterialien,
- keine Verfärbung von Textilien bzw. Flecken sind problemlos auswaschbar,
- Farblosigkeit und Geruchsneutralität,
- wasserfester UV-Schutz.

Die erfindungsgemäßen Verbindungen können als UV-Breitband-Absorber in kosmetischen oder pharmazeutischen Zubereitungen verwendet werden, die den Durchtritt der UV-Strahlen durch den aufgetragenen Film der Zubereitung verhindern. Dies ist im allgemeinen dann der Fall, wenn die kosmetischen bzw. pharmazeutischen Zubereitungen 0,5 bis 15, vorzugsweise 1 bis 10, insbesondere 2 bis 7 Gew.-% (bezogen auf das Gesamtgewicht der Zubereitung) der erfindungsgemäßen Verbindungen enthalten.

Die die erfindungsgemäßen Verbindungen enthaltenden Zubereitungen können zum Schutz der Haut und der Haare - insbesondere durch Dauerwelle, Färbung und Bleichung bereits vorgeschädigter Haare - vor UV-Bestrahlung verwendet werden. Diese zum Schutz der Haut vor der UV-Strahlung dienenden kosmetischen und pharmazeutischen Zubereitungen können in den üblicherweise verwendeten Anwendungsformen vorliegen, d.h. als Öl-in-Wasser- oder Wasser-in-Öl-Emulsion, als Milch, als Lotion oder Creme, wässriges oder wässrig-alkoholisches Gel oder Lotion, Aerosol, Hydrodispersions-Gel (emulgatorfrei) oder jegliche andere übliche kosmetische oder pharmazeutische Zubereitung. Für den Schutz der Haare vor UV-Strahlen werden bevorzugt Zubereitungen als Shampoo, Spülung, Kur, Gel, Lotion, Spray oder Creme verwendet.

Die kosmetischen und pharmazeutischen Zubereitungen können die in diesen Mitteln üblicherweise verwendeten Bestandteile wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropylpalmitat, Isooctylstearat, Adipinsäurediisopropylester usw.), natürliche oder synthetische Öle oder Wachse, Pigmente (z.B. Titandioxid, Zinkoxid, Perglanzpigmente, Farbpigmente), Verdickungsmittel (z.B. Hydroxyethylcellulose, Bentonit usw.), Konservierungsstoffe, Feuchtigkeitsmittel, Vitamine, Siliconöle, Glycerin, Ethylalkohol, Parfumöle enthalten.

Die erfindungsgemäßen Verbindungen können einzeln oder in Mischung in den entsprechenden Zubereitungen eingesetzt werden; man kann sie auch in Kombination mit UV-Absorbern anderer Substanzklassen einsetzen. Beispiele solcher Verbindungen umfassen
p-Aminobenzoesäure
p-Aminobenzoesäureethylester (25 Mol) ethoxyliert
p-Dimethylaminobenzoesäure-2-ethylhexylester
p-Aminobenzoesäurecthylester (2 Mol) N-propoxyliert
p-Aminobenzoesäureglycerinester
Salicylsäure-homomenthylester
Salicylsäure-2-ethylhexylester
Triethanolaminsalicylat
4-Isopropylbenzylsalicylat
Anthranilsäurementhylester
Diisopropylzimtsäureethylester
p-Methoxyzimtsäure-2-ethylhexylester
Diisopropylzimtsäuremethylester
p-Methoxyzimtsäureisoamylester
p-Methoxyzimtsäure-diethanolaminsalz
p-Methoxyzimtsäure-isopropylester
2-Ethylhexyl-2-cyano-3,3-diphenylacrylat

Ethyl-2-cyano-3,3'-diphenylacrylat
2-Phenylbenzimidazolsulfonsäure und Salze
3-(4'-Trimethylammonium)-benzyliden-bornan-2-on-methylsulfat
Terephthalyliden-dibornansulfonsäure und Salze
4-t-Butyl-4'-methoxy-dibenzoylmethan
β-Imidazol-4(5)-acrylsäure (Urocaninsäure)
2-Hydroxy-4-methoxybenzophenon
2-Hydroxy-4-methoxybenzophenon-5-sulfonsäure
Dihydroxy-4-methoxybenzophenon
2,4-Dihydroxybenzophenon
Tetrahydroxybenzophenon
2,2'-Dihydroxy-4,4'-dimethoxybenzophenon
2-Hydroxy-4-n-octoxybenzophenon
2-Hydroxy-4-methoxy-4'-methylbenzophenon
3-(4'-Sulfo)benzyliden-bornan-2-on und Salze
3-(4'-Methylbenzyliden)-d,1-campher
3-Benzyliden-d,1-campher
4-Isopropyldibenzoylmethan
2,4,6-Trianilino-(p-carbo-2'-ethylhexyl-1'-oxy)-1,3,5-triazin
Phenylen-bis-benzimidazyl-tetrasulfonsäure-dinatriumsalz
N-[(2 und 4)-[2-(oxoborn-3-yliden)methyl]benzyl]-acrylamid-Polymer.

Besonders geeignete UV-Absorber sind:

p-Methoxyzimtsäure-2-ethylhexylester,
p-Methoxyzimtsäure-isoamylester,
2-Phenylbenzimidazolsulfonsäure,
3-(4'-Methylbenzyliden)-d,1-campher,
2-Ethylhexyl-2-cyano-3,3-diphenylacrylat,
Salicylsäure-2-ethylhexylester,
4-tert.-Butyl-4'-methoxydibenzoylmethan und
Phenylen-bis-benzimidazyl-tetrasulfonsäure-dinatriumsalz.

Ebenso ist die Kombination der Verbindungen der Formel (I) mit feinteiligen Pigmenten, wie z.B. Titandioxid, Zinkoxid und Eisenoxid, in Sonnenschutz- und Tagespflegeprodukten mit UV-Schutz möglich.

Die erfindungsgemäßen Verbindungen eignen sich auch in besonderem Maße zur Photostabilisierung von UV-Absorbern mit geringer UV-Lichtstabilität. Insbesondere gelingt die Photostabilisierung der sehr lichtinstabilen Verbindungen der Dibenzoylmethane.

Eine lichtstabile UV-Filterkombination zum Schutz der menschlichen Haut gegen UV-Strahlen im Bereich von 280-380 nm in kosmetischen Produkten wird erreicht durch Einsatz von 1 bis 5 Gew.-% von z.B. 4-tert.-Butyl-4'-methoxydibenzoylmethan und mindestens 1 Gew.-% der Verbindung gemäß Formel (I), bevorzugt im Verhältnis 2-4 Teile der Verbindung gemäß Formel (I) zu 1 Teil tert.-Butylmethoxydibenzoylmethan. Das Molverhältnis sollte 1 oder größer sein.

Eine weitere lichtstabile UV-Filterkombination wird erreicht durch Einsatz von 1-10 Gew.-% p-Methoxyzimtsäure-ethylhexylester oder -isoamylester mit mindestens 1 Gew.-% der Verhindung der Formel (I), bevorzugt im Verhältnis 1:1. Das Molverhältnis sollte 0,8 oder größer sein.

Die Kombinationen von p-Methoxyzimtsäureestern und Dibenzoylmethanderivaten und Verbindungen der Formel (I) lassen sich lichtstabil formulieren durch Einsatz von z.B. 1-5 Gew.-% 4-tert.-Butyl-4'-methoxydibenzoylmethan, 1-10 Gew.-% p-Methoxyzimtsäureethylhexyl- oder isoamylester und mindestens 2 Gew.-% der Verbindungen der Formel (I), bevorzugt im Verhältnis 1 Teil Dibenzoylmethanderivat, 2 Teile p-Methoxyzimtsäureester und 2 Teile der Verbindung der Formel (I).

Vorteilhaft ist weiterhin zu dieser Dreierkombination einen weiteren sehr photostabilen UV-Absorber, wie z. B. Methylbenzylidencampher, 2-Ethylhexyl-2-cyano-3,3'-diphenylacrylat oder Octyltriazon, hinzuzusetzen.

Weiterhin lassen sich die erfindungsgemäßen Verbindungen auch mit UV-Absorbem, die für den Schutz technischer Produkte eingesetzt werden, kombinieren.

Beispiele solcher UV-Absorber sind Verbindungen aus der Reihe der Benzotriazole, Benzophenone, Triazine, Zimtsäureester sowie Oxalanilide.

**Beispiele**

**Beispiel 1**

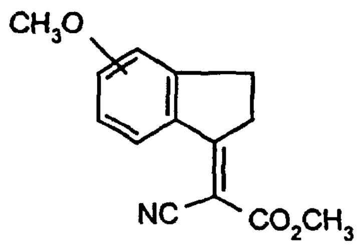

32 g (0,2 mol) 5-Methoxy-1-indanon, 20 g (0,2 mol) Cyanessigsäure-methylester, 17 g Propionsäure und 2 g Ammoniumacetat werden gemischt und auf 120°C 5 Stunden erhitzt. Nach Abkühlen auf Raumtemperatur wird das Rohprodukt aus Methanol umkristallisiert. Ausbeute: 60 % der Theorie; $E^{1/1}$ 1268 ($\lambda_{max}$ 345 nm)

**Beispiel 2**

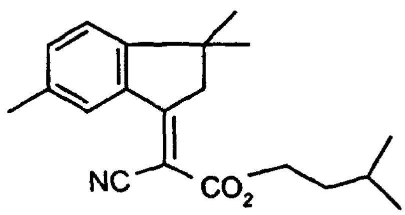

3,3,6-Trimethyl-1-indanon und Cyanessigsäure-isoamylester werden analog Beispiel 1 umgesetzt. Ausbeute: 70 % der Theorie; $E^{1/1}$ 566 ($\lambda_{max}$ 332 nm)/$E^{1/1}$ 551 ($\lambda_{max}$ 309 nm)

**Beispiel 3**

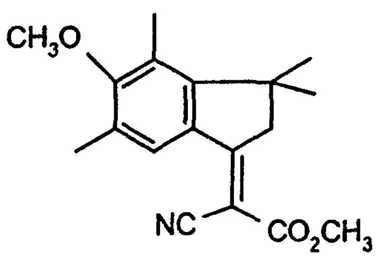

3,3,4,6-Tetramethyl-5-methoxy-1-indanon und Cyanessigsäure-methylester werden analog Beispiel 1 umgesetzt. Ausbeute: 70 % der Theorie; $E^{1/1}$ 800 ($\lambda_{max}$ 338 nm)

**Beispiel 4**

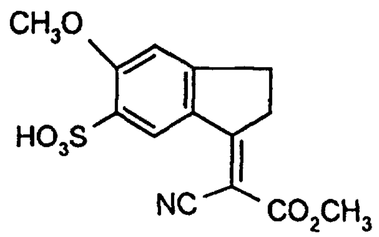

0,1 mol der Verbindung aus Beispiel 1 werden in einem Gemisch aus 0,2 mol Acetanhydrid und 0,2 mol konzentrierter Schwefelsäure 30 Minuten auf 70 bis 80°C erhitzt. Nach Abkühlen auf Raumtemperatur wird das Reaktionsgemisch mit Isopropanol versetzt und das danach ausgefallene Produkt abgesaugt und getrocknet. Ausbeute: 70 % der Theorie; $E^{1/1}$ 800 ($\lambda_{max}$ 345 nm)

| Verwendete Komponenten | | |
|---|---|---|
| Handelsname | Chemische Bezeichnung | Lieferant |
| Abil 100 | Polydimethylsiloxan | 7 |
| Antaron V-216 | Vinylpyrrolidon/Hexadecen Copolymer | 18 |
| Arlacel 1689 | Sorbitan Monooleat/Propylglyceryl-3-ricinoleat | 4 |
| Arlacel 165 | Glycerinstearat/Polyethylenglykol-(MG 100)-Stearat-Mischung | 4 |
| Arlatone G | mit 25 Mol Ethylenoxid gehärtetes | 4 |
| Arlatone 983 S | Polyethylenglykol-(MG 5)-Glycerylstearat | 4 |
| Baysilone Fluid PK 20 | Siliconöl | 5 |
| Betone Gel MIO | Mineralöl, Quaternium-18 Hectorit, Propylencarbonat | 17 |
| Brij 76 | Polyethylenglycol (MG 10) Stearyl-Ether | 4 |
| Carbopol 2984 | Polyacrylsäure | 2 |
| Carbopol 954 | Polyacrylsäure | 2 |
| Cetiol HE | Polyol-Fettsäure-Ester | 3 |
| Cetiol OE | Dicaprylylether | 3 |
| Cetiol SN | Cetyl-/Stearyl-isononanoat | 3 |
| Copherol F 1250 | D-α-Tocopherylacetat | 3 |
| Cutina CBS | Glycerinstearat, Cetyl-/Stearylalkohol, Cetylpalmitat, Kokosnußglyceride | 3 |
| Dehymuls PG PH | Polyglycerinpoly-12-hydroxystearat | 3 |
| Diisopropyladipat | Adipinsäurediisopropylester | 3 |
| D-Panthenol | Panthothenyl-Alkohol | 15 |
| EDTA B fl. | Tetranatrium-ethylendiamintetraessigsäure | 6 |
| Handelsname | Chemische Bezeichnung | Lieferant |
| Eusolex TA | Titandioxid | 13 |
| Eutanol G | 2-Octyldodecanol | 3 |

(fortgesetzt)

| Verwendete Komponenten | | |
|---|---|---|
| Handelsname | Chemische Bezeichnung | Lieferant |
| Eumulgin B2 | Cetyl-/Stearylalkohol, verethert mit 20 Mol Ethylenoxid | 3 |
| Finsolv TN | Alkylbenzoat | 23 |
| Genapol LRO fl. | Natriumlaurylsulfat | 9 |
| Glycerin | 1,2,3-Propantriol | 3 |
| Isopropylmyristat | Myristinsäureisopropylester | 3 |
| Jojobaöl | Jojobaöl - | 19 |
| Lameform TGI | Triglycerindiisostearat | 3 |
| Lamepon S | Eiweiß/Kokosfettsäure-Kondensat, Kaliumsalz | 3 |
| Lanette O | Cetyl-/Stearylalkohol-Mischung | 3 |
| Macadamia-Nußöl | Macadamia-Nußöl | 20 |
| Myritol 318 | Capryl-/Caprin-triglycerid | 3 |
| Natrosol 250 HHR | Hydroxyethylcellulose | 11 |
| NEO HELIOPAN® AV | p-Methoxyzimtsäureisooctylester | 1 |
| NEO HELIOPAN®BB | 2-Hydroxy-4-methoxybenzophenon | 1 |
| NEO HELIOPAN® E 1000 | p-Methoxyzimtsäureisoamylester | 1 |
| NEO HELIOPAN® HYDRO | Phenylbenzimidazolsulfonsäure | 1 |
| NEO HELIOPAN® MBC | 3-(4-Methylbenzylidene)-d, 1-Campher | 1 |
| NEO HELIOPAN®OS | 1-Ethylhexylsalicylat | 1 |
| NEO HELIOPAN® 303 | a-Phenyl-β-cyan-zimtsäureisooctylester | 1 |
| Olivenöl | Olivenöl | 21 |
| Parsol 1789 | Butyl Methoxydibenzoylmethan | 12 |
| Permulgin 2550 | Wachs | 14 |
| Handelsname | Chemische Bezeichnung | Lieferant |
| Permulgin 3220 | Wachs | 14 |
| Permulen TR 1 | Polyacrylat | 2 |
| Phenonip | Gemisch von p-Hydroxybenzoesäureestem und Phenoxyethanol | 8 |
| Polymer JR 400 | Polyquaternium-10 | 21 |
| 1,2-Propylenglykol | 1,2-Propandiol | 6 |
| Texapon MG 3 | Magnesiumlaurylsulfat/Dinatriumlaurylsulfosuccinat | 3 |
| Tocopherolöl | Sojaöl mit D-α-Tocopherol | 22 |
| Uvinul T 150 | Triazinyl-p-aminobenzoesäureisooctylester | 6 |
| Veegum Ultra | Magnesiumaluminiumsilikat | 10 |
| ZINC OXIDE NEUTRAL H&R | Zinkoxid | 1 |
| Zinkstearat | Zinkstearat | 16 |

| Lieferanten | |
|---|---|
| 1. | Haarmann & Reimer GmbH., Holzminden |
| 2. | B.F. Goodrich Company, Neuss |
| 3. | Henkel KGaA, Düsseldorf |
| 4. | ICI Speciality Chemicals, Frankfurt |
| 5. | Bayer AG, Leverkusen |
| 6. | BASF, Ludwigshafen |
| 7. | Goldschmidt AG, Essen |
| 8. | Nipa Lab. Ltd., Pontypridd Mid Glam, Wales / GB |
| 9. | Hoechst AG, Frankfurt |
| 10. | R.T. Vanderbilt Company Inc., Norwalk / USA |
| 11. | Hercules Inc., Wilmington, Delaware/USA |
| 12. | Hoffmann-LaRoche, Basel/CH |
| 13. | E. Merck, Darmstadt |
| 14. | Koster Keunen Holland BV, Bladl /NL |
| 15. | Akzo Chemie GmbH, Düren |
| 16 | Chemische Werke Bärlocher, München |
| 17. | Rheox Inc., Hightstown, New Jersey / USA |
| 18. | ISP Global Technologies Deutschland GmbH, Frechen |
| 19. | Henry Lamotte, Bremen |
| 20 | Erhard Wagner GmbH, Bremen |
| 21. | Nordmann & Rassmann GmbH & Co., Hamburg |
| 22 | Richter GmbH, Berlin |
| 23. | Witco Surfactants GmbH, Steinau a.d. Straße |

**Beispiel 5**

| Sonnenschutzlotion (O/W) | | |
|---|---|---|
| | BESTANDTEILE | % |
| A) | Arlatone 983 S | 1,75 |
| | Brij 76 | 1,25 |
| | Lanette O | 1,15 |
| | Myritol 318 | 5,00 |
| | Eutanol G | 6,00 |
| | Cetiol SN | 6,00 |
| | Phenonip | 0,20 |
| | UV-Absorber gemäß Formel (I) | 3,00 |
| B) | Wasser, dest. | 46,65 |
| | 1,2-Propylenglykol | 2,00 |

(fortgesetzt)

| Sonnenschutzlotion (O/W) | | |
|---|---|---|
| | BESTANDTEILE | % |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfümöl | 0,40 |
| HERSTELLVORSCHRIFT:<br>Teil A: Bei ca. 80°C aufschmelzen<br>Teil B: Auf ca. 90°C erhitzen, Teil B unter Rühren zu Teil A geben.<br>Teil C: Carbopol klumpenfrei in Wasser dispergieren, mit Natriumhydroxid-Lösung zu einem Gel neutralisieren, bei ca. 60°C zu Teil A/B geben.<br>Auf Raumtemperatur abrühren.<br>Teil D: Bei ca. 30°C die Emulsion parfümieren, pH-Wert kontrollieren (6,5 bis 7.0). | | |

**Beispiel 6**

| Sonnenschutzlotion (O/W) | | |
|---|---|---|
| | BESTANDTEILE | % |
| A) | Arlatone 983 S | 1,75 |
| | Brij 76 | 1,25 |
| | Lanette O | 1,15 |
| | Myritol 318 | 15,00 |
| | Cetiol SN | 15,00 |
| | Phenonip | 0,20 |
| | UV-Absorber gemäß Formel (I) | 5,00 |
| B) | Wasser, dest. | 31,65 |
| | 1,2-Propylenglykol | 2,00 |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfümöl | 0,40 |
| HERSTELLVORSCHRIFT:<br>Teil A: Bei ca. 80°C aufschmelzen<br>Teil B: Auf ca. 90°C erhitzen, Teil B unter Rühren zu Teil A geben.<br>TeilC: Carbopol klumpenfrei in Wasser dispergieren, mit Natriumhydroxid-Lösung zu einem Gel neutralisieren, bei ca. 60°C zu Teil A/B geben.<br>Auf Raumtemperatur abrühren.<br>Teil D: Bei ca. 30°C die Emulsion parfümieren, pH-Wert kontrollieren (6,5 bis 7,0). | | |

Die Bestimmung des in-vitro-Lichtschutzfaktors in Anlehnung an die Methode von Diffey u. Robson ("A new substrate to measure sunscreen protection factors throughout the ultra violet spectrum," J. Soc. Cosm. Chem. 40 (3),

123-133 (1989)) ergab einen Wert von 11,0.

**Beispiel 7**

| Sonnenschutzmilch (W/O) | | |
|---|---|---|
| | BESTANDTEILE | % |
| A) | Dehymuls PG PH | 5,00 |
| | Permulgin 3220 | 0,50 |
| | Zinkstearat | 0,50 |
| | Myritol 318 | 15,00 |
| | Cetiol SN | 15,00 |
| | UV-Absorber gemäß Formel (I) | 5,00 |
| B) | Wasser, dest. | 52,50 |
| | Glycerin 86 % | 5,00 |
| | Magnesiumsulfat 7 $H_2O$ | 0,50 |
| | Phenonip | 0,50 |
| C) | Parfumöl | 0,50 |

HERSTELLVORSCHRIFT:

Teil A: Bei ca. 90°C sorgfältig aufschmelzen.

Teil B: Auf ca. 95°C erhitzen, dann Teil B unter Rühren zu Teil A geben. Auf Raumtemperatur abrühren.

Teil C: Bei 30°C Teil C zugeben und anschließend homogenisieren.

Die Bestimmung des in-vitro-Lichtschutzfaktors in Anlehnung an die Methode von Diffey u. Robson ("A new substrate to measure sunscreen protection factors throughout the ultra violet spectrum," J. Soc. Cosm. Chcm. 40 (3). 123-133 (1989)) ergab einen Wert von 11,9.

**Beispiel 8**

| Sonnenschutzlotion (O/W) | | |
|---|---|---|
| | BESTANDTEILE | % |
| A) | Ariatone 983 S | 1,75 |
| | Brij 76 | 1,25 |
| | Lanette O | 1,15 |
| | Myritol 318 | 15,00 |
| | Cetiol SN | 15,00 |
| | Finsolv TN | 5,00 |
| | Phenonip | 0,20 |
| | UV-Absorber gemäß Formel (I) | 4,00 |
| | Parsol 1789 | 1,50 |
| B) | Wasser, dest. | 26,15 |
| | 1,2-Propylenglykol | 2,00 |

(fortgesetzt)

| Sonnenschutzlotion (O/W) | | |
|---|---|---|
| | BESTANDTEILE | % |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfumöl | 0,40 |

HERSTELLVORSCHRIFT:

Teil A: Bei ca. 80°C aufschmelzen

Teil B: Auf ca. 90°C erhitzen. Teil B unter Rühren zu Teil A geben.

Teil C: Carbopol klumpenfrei in Wasser dispergieren, mit Natriumhyroxid-Lösung zu einem Gel neutralisieren, bei ca. 60°C zu Teil A/B geben. Auf Raumtemperatur abrühren.

Teil D: Bei ca. 30°C die Emulsion parfümieren, pH-Wert kontrollieren (6,5 bis 7,0).

**Beispiel 9**

| Sonnenschutzlotion (O/W) | | |
|---|---|---|
| | BESTANDTEILE | % |
| A) | Ariatone 983 S | 1,75 |
| | Brij 76 | 1,25 |
| | Lanette O | 1,15 |
| | Myritol 318 | 12,00 |
| | Cetiol SN | 12,00 |
| | UV-Absorber gemäß Formel (I) | 7,00 |
| | NEO HELIOPAN® E 1000 | 7,00 |
| | Phenonip | 0,20 |
| B) | Wasser, dest. | 28,65 |
| | 1,2-Propylenglykol | 2,00 |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfumöl | 0,40 |

HERSTELLVORSCHRIFT:

Teil A: Bei ca. 80°C aufschmelzen

Teil B: Auf ca. 90°C erhitzen. Teil B unter Rühren zu Teil A geben.

Teil C: Carbopol klumpenfrei in Wasser dispergieren, mit Natriumhyroxid-Lösung zu einem Gel neutralisieren, bei ca. 60°C zu Teil A/B geben. Auf Raumtemperatur abrühren.

Teil D: Bei ca. 30°C die Emulsion parfümieren, pH-Wert kontrollieren (6,5 bis 7,0).

**Beispiel 10**

| Sonnenschutzlotion (O/W) | | |
|---|---|---|
| | BESTANDTEILE | % |
| A) | Arlacel 165 | 3,00 |
| | Eumulgin B 2 | 1,00 |
| | Lanette | 1,00 |
| | Myritol 318 | 4,00 |
| | Cetiol OE | 2,00 |
| | Abil 100 | 1,00 |
| | Bentone Gel MIO | 3,00 |
| | Cutina CBS | 1,00 |
| | Phenonip | 0,20 |
| | NEO HELIOPAN® OS (Octyl Salicylate) | 3,00 |
| | NEO HELIOPAN® AV (Octyl Methoxycinnamate) | 5,00 |
| | NEO HELIOPAN® E 1000 (Isoamyl p-Methoxycinnamate) | 5,00 |
| | NEOHELIOPAN® MBC (4-Methylbenzylidene Camphor) | 1,00 |
| | Eusolex TA | 3,00 |
| | UV-Absorber gemäß Formel (I) | 3,00 |
| B) | Wasser, dest. | 45,60 |
| | Glycerin, 86%ig | 3,00 |
| | Phenonip | 0,30 |
| | Veegum Ultra | 1,00 |
| | Natrosol 250 HHR | 0,30 |
| | BESTANDTEILE<br>NEO HELIOPAN ® HYDRO, eingesetzt als 15%ige Lösung nach Neutralisation mit Natriumhydroxid (Phenyl benzimidazole Sulfonic Acid) entspricht Aktivsubstanz: 2,0% | %<br>13,30 |
| C) | Pafümöl | 0,30 |
| HERSTELLVORSCHRIFT:<br>Teil A: Bei ca. 80°C aufschmelzen, dann Eusolex TA sorgfältig dispergieren.<br>Teil B: Ohne Veegum und Natrosol auf ca. 90°C erhitzen, dann Veegum und Natrosol dispergieren, Teil B unter Rühren zu Teil A geben. Auf Raumtemperatur abrühren.<br>Teil C: Bei 30°C Teil C zugeben und anschließend homogenisieren. pH-Wert überprüfen (7,0-7,5). | | |

**Beispiel 11**

| Sonnenschutzlotion (W/O) | | |
|---|---|---|
| | BESTANDTEILE | % |
| A) | Arlacel 1689 | 3,50 |
| | Finsolv TN | 6,00 |

(fortgesetzt)

| Sonnenschutzlotion (W/O) | | |
|---|---|---|
| | BESTANDTEILE | % |
| | NEO HELIOPAN® E 1000 (Isoamyl p-Methoxycinnamate) | 7,00 |
| | Uvinul T 150 (Octyl Triazone) | 1,00 |
| | UV-Absorber gemäß Formel (I) | 3,00 |
| | Copherol F 1250 | 2,00 |
| | Permulgin 2550 | 1,00 |
| | Myritol 318 | 6,00 |
| | Cetiol SN | 6,00 |
| | ZINKOXID NEUTRAL H&R (Zinc Oxide) | 7,00 |
| B) | Wasser, dest. | 51,70 |
| | Glycerin 86% | 5,00 |
| | Phenonip | 0,50 |
| C) | Parfumöl | 0,30 |

HERSTELLVORSCHRIFT:

Teil A: Bei ca. 90°C sorgfältig aufschmelzen (ohne ZINKOXID NEUTRAL H&R). Anschließend ZINKOXID NEUTRAL H&R sorfältig dispergieren.

Teil B: Auf ca. 95°C erhitzen, dann Teil B unter Rühren zu Teil A geben. Auf Raumtemperatur abrühren.

Teil C: Bei 30°C Teil C zugeben und anschließend homogenisieren.

**Beispiel 12**

| Sonnenschutzöl | | |
|---|---|---|
| | BESTANDTEILE | % |
| A) | NEO HELIOPAN® E 1000 (Isoamyl p-Methoxycinnamate) | 7,50 |
| | NEO HELIOPAN® OS (Octyl Salicylate) | 5,00 |
| | UV-Absorber gemäß Formel (I) | 3,00 |
| | Myritol 318 | 34,70 |
| | Diisopropyladipat | 5,00 |
| | Olivenöl | 1,00 |
| | Jojobaöl | 1,00 |
| | Macadamia Nußöl | 1,00 |
| | Tocopherolöl | 1,00 |
| | Isopropylmyristat | 35,00 |
| | Antaron V-216 | 5,00 |
| | Phenonip | 0,50 |
| | Parfumöl | 0,30 |

HERSTELLVORSCHRIFT:

Alle Bestandteile sorfältig vermischen.

**Beispiel 13**

| Sonnenschutz Creme Gel | | |
|---|---|---|
| | BESTANDTEILE | % |
| A) | Wasser, dest. | 75,35 |
| | Phenonip | 0,50 |
| | EDTA B liquid | 0,10 |
| B) | NEO HELIOPAN® AV (Octyl Methoxycinnamate) | 7,00 |
| | NEO HELIOPAN® 303 (Octocrylene) | 3,00 |
| | NEO HELIOPAN® MBC (4-Methylbenzylidene Camphor) | 1,00 |
| | UV-Absorber gemäß Formel (I) | 3,00 |
| | Cetiol SN | 5,00 |
| | Eutanol G | 3,00 |
| | Lameform TG I | 1,00 |
| | Parfümöl | 0,30 |
| | Permulen TR-1 | 0,25 |
| | Carbopol 954 | 0,05 |
| C) | Triethanolamin | 0,45 |
| HERSTELLVORSCHRIFT: Teil A: Inhaltstoffe im Wasser lösen. Teil B: Alle Bestandteile (ohne Permulen und Carbopol) vermischen. NEO HELIOPAN® MBC und UV-Absorber gemäß Formel (I) unter leichtem Erwärmen lösen. Carbopol und Permulen dispergieren. Anschließend Teil B zu Teil A geben und 45 Minuten intensiv verrühren. Teil C: Triethanolamin unter Rühren zu Teil A/B geben. Weiterrühren bis das Produkt homogen ist. pH Wert kontrollieren (ca. 7,0). | | |

**Beispiel 14**

| **Haarschampoo** | | |
|---|---|---|
| | BESTANDTEILE | % |
| A) | Genapol LRO flüssig | 18,00 |
| | Texapon MG3 | 36,00 |
| | Lamepon S | 6,00 |
| | Parfumöl | 0,60 |
| | Phenonip | 0,50 |
| | Arlatone G | 2,00 |
| | UV-Absorber gemäß Formel (I) | 0,50 |
| | NEO HELIOPAN® E 1000 (Isoamyl p-Methoxycinnamate) | 1,00 |
| B) | Wasser, dest. | 33,00 |
| | Polymer JR 400 | 0,20 |

(fortgesetzt)

| Haarschampoo | | |
|---|---|---|
| | BESTANDTEILE | % |
| | D-Panthenol | 1,00 |
| | Natriumchlorid | 1,00 |
| | Natriumhydroxid, 10%ig in Wasser | 0,20 |

HERSTELLVORSCHRIFT:

Teil A: UV-Absorber in NEO HELIOPAN® E 1000 und Phenonip unter schwachem Erwärmen lösen, dann Arlatone G und Parfumöl zugeben und gut vermischen. Restliche Bestandteile einwiegen.

Teil B: Polymer under Rühren im Wasser lösen, restliche Bestandteile zugeben und lösen. Teil B zu Teil A geben und verrühren (pH-Wert kontrollieren, ca. 5,5).

**Patentansprüche**

**1.** Kosmetische und pharmazeutische Zubereitungen enthaltend Indanyliden-Verbindungen der Formel (I)

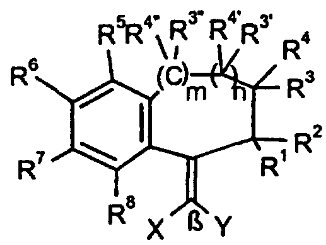

(I)

worin

| | |
|---|---|
| $R^1$ bis $R^4$, $R^{3'}$, $R^{3''}$, $R^{4'}$ und $R^{4''}$ | unabhängig voneinander Wasserstoff, $C_1$-$C_8$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe, insbesondere $C_3$-$C_4$-Alkylen, bedeuten können, wobei eine Methylengruppe durch -O-, -S-, oder -NH- ersetzt sein kann, weiterhin unabhängig voneinander $C_1$-$C_4$-Alkoxy, Hydroxy, Carboxy, Carbalkoxy oder Carbamoyl bedeuten, |
| $R^5$ bis $R^8$ | unabhängig voneinander die Bedeutung von $R^1$, $R^2$ oder Sulfo oder Aminosulfonyl haben, |
| X und Y | unabhängig voneinander CN, $CO_2R^9$, $CONR^9R^{10}$ oder $COR^9$ bedeuten, wobei $R^9$ und $R^{10}$ unabhängig voneinander für Wasserstoff, $C_1$ bis $C_8$-Alkyl oder $C_5$-$C_{10}$-Cycloalkyl stehen und weiterhin einer der Reste, |
| X oder Y | zusätzlich ein $C_1$-$C_8$-Alkylrest, ein $C_5$-$C_{10}$-Arylrest, insbesondere Phenyl, oder ein 5- bis 6-gliedriger Heteroarylrest welcher 1 bis 2 Heteroatome aus der Reihe N, O, S enthält sein kann, oder |
| X und Y | zusammen mit dem β-Atom, an welches sie gebunden sind, einen 5-bis 7-gliedrigen Ring bedeuten, der bis zu 3 Heteroatome, insbesondere Sauerstoff und/oder Stickstoff enthält, wobei die Ringatome substituiert sein können, insbesondere mit exocyclisch doppelt gebundenem Sauerstoff (Ketogruppe), bevorzugt in Nachbarstellung zu dem β-Atom, und |
| n, m | unabhängig voneinander Null oder 1 bedeuten, |

als UV-Absorber.

**2.** Kosmetische und pharmazeutische Zubereitungen nach Anspruch 1 enthaltend Indanyliden-Verbindungen nach Anspruch 1 der Formel (I)

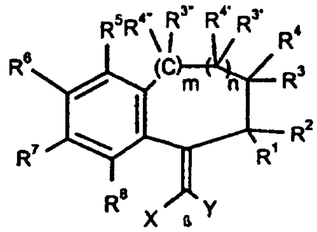

(I)

worin

$R^1$ bis $R^4$, $R^{3'}$, $R^{3''}$, $R^{4'}$ und $R^{4''}$ unabhängig voneinander Wasserstoff, $C_1$-$C_8$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe, insbesondere $C_3$-$C_4$-Alkylen, bedeuten können, wobei eine Methylengruppe durch -O-, -S-, oder -NH- ersetzt sein kann, weiterhin unabhängig voneinander $C_1$-$C_4$-Alkoxy, Hydroxy, Carboxy, Carbalkoxy oder Carbamoyl bedeuten,

$R^5$ bis $R^8$ unabhängig voneinander die Bedeutung von $R^1$, $R^2$ oder Sulfo oder Aminosulfonyl haben,

X Cyan und

Y Carb-$C_1$-$C_4$-Alkoxy bedeuten.

**3.** Kosmetische und pharmazeutische Zubereitungen nach den Ansprüchen 1 und 2 enthaltend Indanyliden-Verbindungen der Formeln

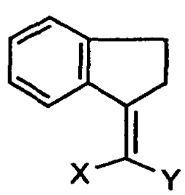

(Ia)

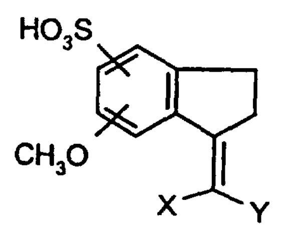

(Ib)

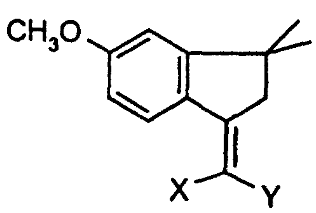

(Ic)

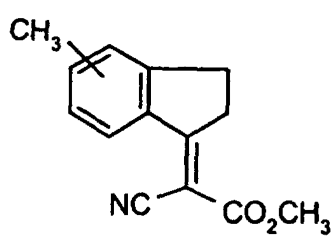

(Id)

worin

X Cyan und

Y Carb-$C_1$-$C_4$-Alkoxy bedeuten.

**4.** Kosmetische oder pharmazeutische Zubereitungen nach den Ansprüchen 1 bis 3 enthaltend 0,5 bis 15 Gew.-% der Indanyliden-Verbindungen bezogen auf das Gesamtgewicht der Zubereitung.

**5.** Kosmetische und pharmazeutische Zubereitungen nach den Ansprüchen 1 bis 4 enthaltend 1 bis 5 Gew.-% 4-tert. -Butyl-4-methoxydibenzoylmethan und mindestens 1 Gew.-% der Indanyliden-Verbindung bezogen auf das Gesamtgewicht der Zubereitung.

**6.** Kosmetische und pharmazeutische Zubereitungen nach den Ansprüchen 1 bis 4 enthaltend 1 bis 10 Gew.-% p-Methoxyzimtsäure-ethylhexylester oder p-Methoxyzimtsäure-isoamylester und mindestens 1 Gew.-% der Indanyliden-Verbindung bezogen auf das Gesamtgewicht der Zubereitung.

**7.** Verwendung von kosmetischen und pharmazeutischen Zubereitungen nach den Ansprüchen 4 bis 6 zum Schutz der Haut und der Haare vor UV-Bestrahlung.

**8.** Verwendung von Indanyliden-Verbindungen der Formel (I)

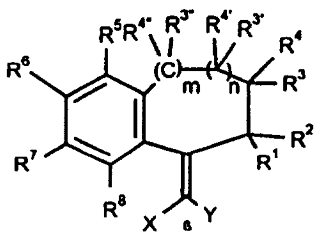

(I)

worin

| | |
|---|---|
| $R^1$ bis $R^4$, $R^{3'}$, $R^{3''}$, $R^{4'}$ und $R^{4''}$ | unabhängig voneinander Wasserstoff, $C_1$-$C_8$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe, insbesondere $C_3$-$C_4$-Alkylen, bedeuten können, wobei eine Methylengruppe durch -O-, -S-, oder -NH- ersetzt sein kann, weiterhin unabhängig voneinander $C_1$-$C_4$-Alkoxy, Hydroxy, Carboxy, Carbalkoxy oder Carbamoyl bedeuten, |
| $R^5$ bis $R^8$ | unabhängig voneinander die Bedeutung von $R^1$, $R^2$ oder Sulfo oder Aminosulfonyl haben, |
| X und Y | unabhängig voneinander CN, $CO_2R^9$, $CONR^9R^{10}$ oder $COR^9$ bedeuten, wobei $R^9$ und $R^{10}$ unabhängig voneinander für Wasserstoff, $C_1$ bis $C_8$-Alkyl oder $C_5$-$C_{10}$-Cycloalkyl stehen und weiterhin einer der Reste, |
| X oder Y | zusätzlich ein $C_1$-$C_8$-Alkylrest, ein $C_5$-$C_{10}$-Arylrest, insbesondere Phenyl, oder ein 5- bis 6-gliedriger Heteroarylrest welcher 1 bis 2 Heteroatome aus der Reihe N, O, S enthält sein kann, oder |
| X und Y | zusammen mit dem β-Atom, an welches sie gebunden sind, einen 5-bis 7-gliedrigen Ring bedeuten, der bis zu 3 Heteroatome, insbesondere Sauerstoff und/oder Stickstoff enthält, wobei die Ringatome substituiert sein können, insbesondere mit exocyclisch doppelt gebundenem Sauerstoff (Ketogruppe), bevorzugt in Nachbarstellung zu dem β-Atom, |

und

n, m  unabhängig voneinander Null oder 1 bedeuten,

als UV-Absorber.

**9.** Verwendung nach Anspruch 8 als UV-Absorber in kosmetischen oder pharmazeutischen Zubereitungen.

**10.** Verwendung nach den Ansprüchen 8 und 9 als UV-Absorber in Sonnenschutzmitteln.

**11.** Verwendung nach den Ansprüchen 8 bis 10 als UV-Absorber in Kombination mit feinteiligen Pigmenten in Sonnenschutz- und Tagespflegeprodukten.

**12.** Indanylidenverbindungen der Formel (I)

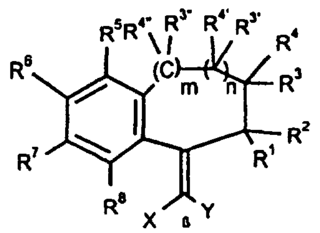

(I)

worin

| | |
|---|---|
| $R^1$ bis $R^4$, $R^{3'}$, $R^{3''}$, $R^{4'}$ und $R^{4''}$ | unabhängig voneinander Wasserstoff, $C_1$-$C_8$-Alkyl oder $C_5$-$C_{10}$-Cycloalkyl bedeuten, mit der Maßgabe, dass auch zwei Substituenten an benachbarten C-Atomen zusammen eine gegebenenfalls substituierte $C_1$-$C_4$-Alkylengruppe, insbesondere $C_3$-$C_4$-Alkylen, bedeuten können, wobei eine Methylengruppe durch -O-, -S-, oder -NH- ersetzt sein kann, weiterhin unabhängig voneinander $C_1$-$C_4$-Alkoxy, Hydroxy, Carboxy, Carbalkoxy oder Carbamoyl bedeuten, |
| $R^5$ bis $R^8$ | unabhängig voneinander die Bedeutung von $R^1$, $R^2$ oder Sulfo oder Aminosulfonyl haben, |
| X und Y | unabhängig voneinander CN, $CO_2R^9$, $CONR^9R^{10}$ oder $COR^9$ bedeuten, wobei $R^9$ und $R^{10}$ unabhängig voneinander für Wasserstoff, $C_1$ bis $C_8$-Alkyl oder $C_5$-$C_{10}$-Cycloalkyl stehen und weiterhin einer der Reste, |
| X oder Y | zusätzlich ein $C_1$-$C_8$-Alkylrest, ein $C_5$-$C_{10}$-Arylrest, insbesondere Phenyl, oder ein 5- bis 6-gliedriger Heteroarylrest welcher 1 bis 2 Heteroatome aus der Reihe N, O, S enthält sein kann, oder |
| X und Y | zusammen mit dem β-Atom, an welches sie gebunden sind, einen 5-bis 7-gliedrigen Ring bedeuten, der bis zu 3 Heteroatome, insbesondere Sauerstoff und/oder Stickstoff enthält, wobei die Ringatome substituiert sein können, insbesondere mit exocyclisch doppelt gebundenem Sauerstoff (Ketogruppe), bevorzugt in Nachbarstellung zu dem β-Atom, |

und

n, m  unabhängig voneinander Null oder 1 bedeuten,

wobei die Verbindungen der Formeln

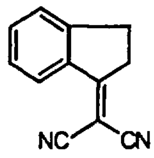
I

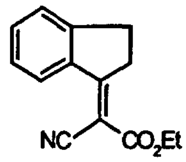
II

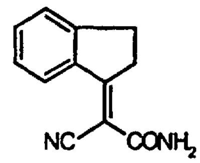
III

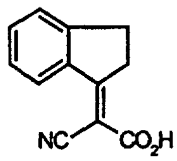
IV

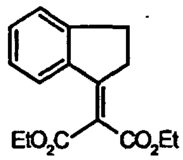
V

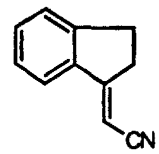
VI

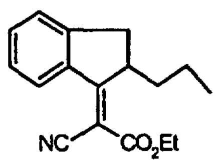
VII

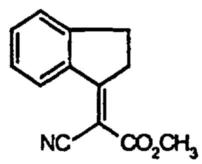
VIII

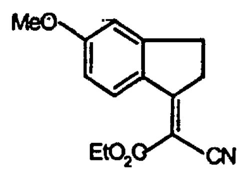
IX

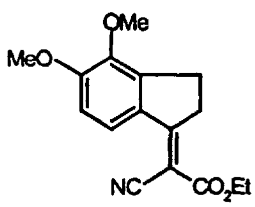
X

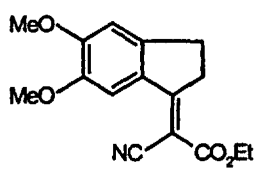
XI

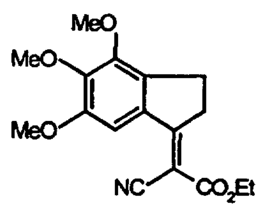
XII

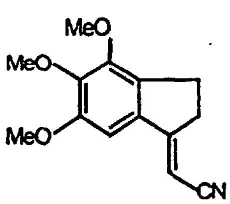
XIII

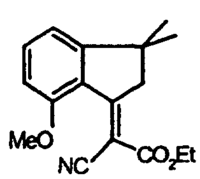
XIV

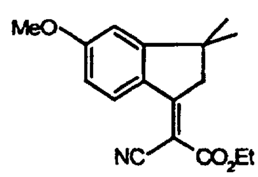
XV

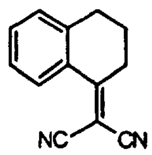
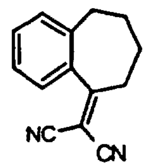

XVI XVII

sowie der allgemeinen Formel

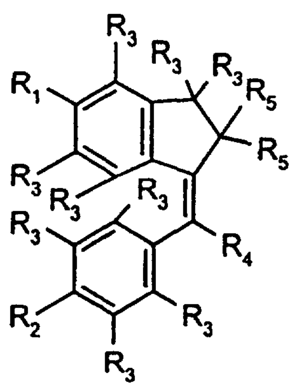

worin

$R_1$, $R_2$ und $R_3$ jeweils unabhängig voneinander gleich oder verschieden sein können und Wasserstoffatom, Hydroxygruppe oder einwertiger, gegebenenfalls substituierter organischer Rest bedeuten und

$R_4$ Cyanogruppe oder einwertiger, gegebenenfalls substituierter organischer Rest bedeutet und

$R_5$ gleich oder verschieden sein kann und Hydroxygruppe oder einwertiger, gegebenenfalls substituierter organischer Rest bedeutet,

ausgenommen sind.

**13.** Verbindungen der Formel (Ib)

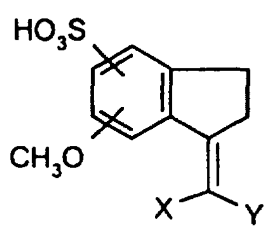

(Ib)

wobei

X und Y unabhängig voneinander CN, $CO_2R^9$, $CONR^9R^{10}$ oder $COR^9$ bedeuten, wobei $R^9$ und $R^{10}$ unabhängig voneinander für Wasserstoff, $C_1$ bis $C_8$-Alkyl oder $C_5$-$C_{10}$-Cycloalkyl stehen und weiterhin einer der Reste,

X oder Y zusätzlich ein $C_1$-$C_8$-Alkylrest, ein $C_5$-$C_{10}$-Arylrest, insbesondere Phenyl, oder ein 5- bis 6-gliedriger

Heteroarylrest welcher 1 bis 2 Heteroatome aus der Reihe N, O, S enthält sein kann, oder

X und Y zusammen mit dem β-Atom, an welches sie gebunden sind, einen 5-bis 7-gliedrigen Ring bedeuten, der bis zu 3 Heteroatome, insbesondere Sauerstoff und/oder Stickstoff enthält, wobei die Ringatome substituiert sein können, insbesondere mit exocyclisch doppelt gebundenem Sauerstoff (Ketogruppe), bevorzugt in Nachbarstellung zu dem β-Atom,

bedeuten.

## Claims

1. Cosmetic and pharmaceutical preparations containing indanylidene compounds having the formula (I)

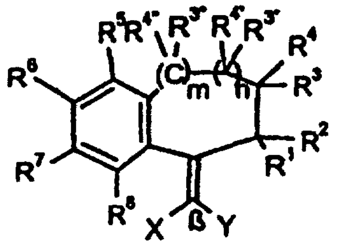

(I)

wherein

| | |
|---|---|
| $R^1$ to $R^4$, $R^{3'}$. $R^{3''}$, $R^{4'}$ and $R^{4''}$ | mutually independently denote hydrogen, $C_1$-$C_8$ alkyl or $C_5$-$C_{10}$ cycloalkyl, with the proviso that two substituents on adjacent C atoms can together also denote an optionally substituted $C_1$-$C_4$ alkylene group, in particular $C_3$-$C_4$ alkylene, whereby a methylene group can be replaced by -O-. -S- or -NH-, and furthermore mutually independently denote $C_1$-$C_4$ alkoxy, hydroxy, carboxy, carbalkoxy or carbamoyl, |
| $R^5$ to $R^8$ | mutually independently have the meaning of $R^1$, $R^2$ or sulfo or aminosulfonyl, |
| X and Y | mutually independently denote CN, $CO_2R^9$, $CONR^9R^{10}$ or $COR^9$, whereby $R^9$ and $R^{10}$ mutually independently stand for hydrogen, $C_1$ to $C_8$ alkyl or $C_5$ to $C_{10}$ cycloalkyl and furthermore one of the radicals, |
| X or Y | can additionally be a $C_1$ to $C_8$ alkyl radical, a $C_5$ to $C_{10}$ aryl radical, in particular phenyl, or a 5- to 6-membered heteroaryl radical containing 1 to 2 heteroatoms from the series N, O, S, or |
| X and Y | together with the β-atom to which they are bonded denote a 5- to 7-membered ring containing up to 3 heteroatoms, in particular oxygen and/or nitrogen, whereby the ring atoms can be substituted, particularly with exocyclically double-bonded oxygen (keto group) preferably in a vicinal position to the β-atom, |

and

n, m mutually independently denote zero or 1,

as UV absorbers.

2. Cosmetic and pharmaceutical preparations according to claim I containing indanylidene compounds according to claim 1 having formula (I)

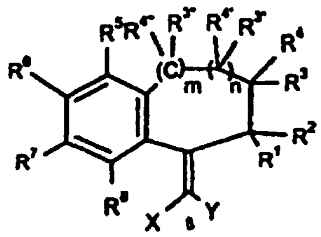

(I)

wherein

| | |
|---|---|
| $R^1$ to $R^4$, $R^{3'}$. $R^{3''}$, $R^{4'}$ and $R^{4''}$ | mutually independently denote hydrogen, $C_1$-$C_8$ alkyl or $C_5$-$C_{10}$ cycloalkyl, with the proviso that two substituents on adjacent C atoms can together also denote an optionally substituted $C_1$-$C_4$ alkylene group, in particular $C_3$-$C_4$ alkylene, whereby a methylene group can be replaced by -O-. -S- or -NH-, and furthermore mutually independently denote $C_1$-$C_4$ alkoxy, hydroxy, carboxy, carbalkoxy or carbamoyl, |
| $R^5$ to $R^8$ | mutually independently have the meaning of $R^1$, $R^2$ or sulfo or aminosulfonyl, |
| X | denotes cyanogen and |
| Y | denotes carb-$C_1$-$C_4$-alkoxy. |

**3.** Cosmetic and pharmaceutical preparations according to claims 1 and 2 containing indanylidene compounds having the formulae

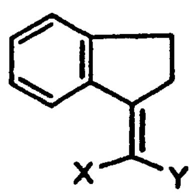

(Ia)

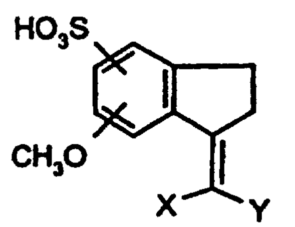

(Ib)

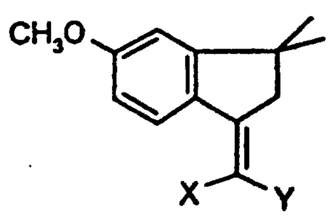

(Ic)

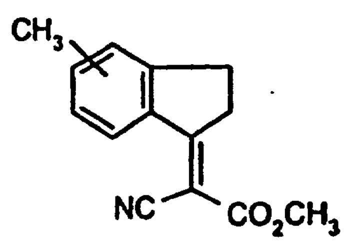

(Id)

wherein

X denotes cyanogen and

Y denotes carb-$C_1$-$C_4$-alkoxy.

**4.** Cosmetic or pharmaceutical preparations according to claims 1 to 3 containing 0.5 to 15 wt.% of indanylidene compounds relative to the overall weight of the preparation.

**5.** Cosmetic and pharmaceutical preparations according to claims 1 to 4 containing 1 to 5 wt.% 4-tert.-butyl-4-methoxydibenzoyl methane and at least 1 wt.% of the indanylidene compound relative to the overall weight of the preparation.

**6.** Cosmetic and pharmaceutical preparations according to claims 1 to 4 containing 1 to 10 wt.% p-methoxycinnamic acid ethyl hexyl ester or p-methoxycinnamic acid isoamyl ester and at least 1 wt.% of the indanylidene compound relative to the overall weight of the preparation.

**7.** Use of cosmetic and pharmaceutical preparations according to claims 4 to 6 for protecting the skin and hair against UV radiation.

**8.** Use of indanylidene compounds having the formula (I)

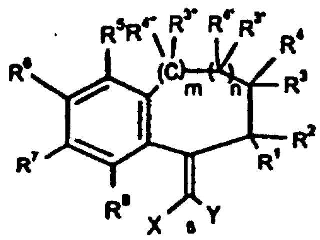

(I)

wherein

| | |
|---|---|
| $R^1$ to $R^4$, $R^{3'}$. $R^{3''}$, $R^{4'}$ and $R^{4''}$ | mutually independently denote hydrogen, $C_1$-$C_8$ alkyl or $C_5$-$C_{10}$ cycloalkyl, with the proviso that two substituents on adjacent C atoms can together also denote an optionally substituted $C_1$-$C_4$ alkylene group, in particular $C_3$-$C_4$ alkylene, whereby a methylene group can be replaced by -O-. -S- or -NH-, and furthermore mutually independently denote $C_1$-$C_4$ alkoxy, hydroxy, carboxy, carbalkoxy or carbamoyl, |
| $R^5$ to $R^8$ | mutually independently have the meaning of $R^1$, $R^2$ or sulfo or aminosulfonyl, |
| X and Y | mutually independently denote CN, $CO_2R^9$, $CONR^9R^{10}$ or $COR^9$, whereby $R^9$ and $R^{10}$ mutually independently stand for hydrogen, $C_1$ to $C_8$ alkyl or $C_5$ to $C_{10}$ cycloalkyl and furthermore one of the radicals, |
| X or Y | can additionally be a $C_1$ to $C_8$ alkyl radical, a $C_5$ to $C_{10}$ aryl radical, in particular phenyl, or a 5- to 6-membered heteroaryl radical containing 1 to 2 heteroatoms from the series N, O, S, or |
| X and Y | together with the β-atom to which they are bonded denote a 5- to 7-membered ring containing up to 3 heteroatoms, in particular oxygen and/or nitrogen, whereby the ring atoms can be substituted, particularly with exocyclically double-bonded oxygen (keto group) preferably in a vicinal position to the β-atom, |

and

n, m mutually independently denote zero or 1,

as UV absorbers.

**9.** Use according to claim 8 as a UV absorber in cosmetic or pharmaceutical preparations.

**10.** Use according to claims 8 and 9 as a UV absorber in sunscreens.

**11.** Use according to claims 8 to 10 as a UV absorber in combination with fine-particle pigments in sunscreen and daily care products.

**12.** Indanylidene compounds having the formula (I)

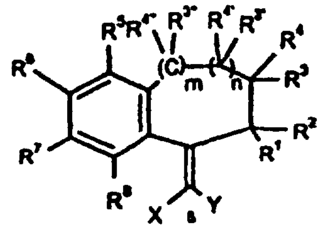

(I)

wherein

| | |
|---|---|
| $R^1$ to $R^4$, $R^{3'}$. $R^{3''}$, $R^{4'}$ and $R^{4''}$ | mutually independently denote hydrogen, $C_1$-$C_8$ alkyl or $C_5$-$C_{10}$ cycloalkyl, with the proviso that two substituents on adjacent C atoms can together also denote an optionally substituted $C_1$-$C_4$ alkylene group, in particular $C_3$-$C_4$ alkylene, whereby a methylene group can be replaced by -O-. -S- or -NH-, and furthermore mutually independently denote $C_1$-$C_4$ alkoxy, hydroxy, carboxy, carbalkoxy or carbamoyl, |
| $R^5$ to $R^8$ | mutually independently have the meaning of $R^1$, $R^2$ or sulfo or aminosulfonyl, |
| X and Y | mutually independently denote CN, $CO_2R^9$, $CONR^9R^{10}$ or $COR^9$, whereby $R^9$ and $R^{10}$ mutually independently stand for hydrogen, $C_1$ to $C_8$ alkyl or $C_5$ to $C_{10}$ cycloalkyl and furthermore one of the radicals, |
| X or Y | can additionally be a $C_1$ to $C_8$ alkyl radical, a $C_5$ to $C_{10}$ aryl radical, in particular phenyl, or a 5- to 6-membered heteroaryl radical containing 1 to 2 heteroatoms from the series N, O, S, or |
| X and Y | together with the β-atom to which they are bonded denote a 5- to 7-membered ring containing up to 3 heteroatoms, in particular oxygen and/or nitrogen, whereby the ring atoms can be substituted, particularly with exocyclically double-bonded oxygen (keto group) preferably in a vicinal position to the β-atom, |

and

n, m mutually independently denote zero or 1,

whereby the compounds having the formulae

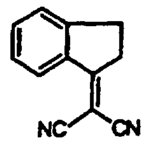

I

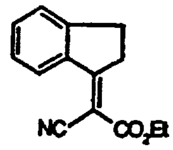

II

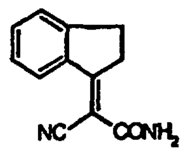

III

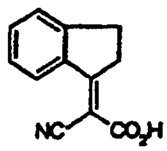

IV

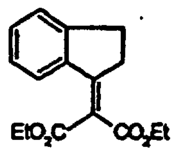

V

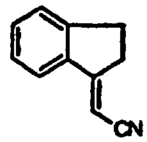

VI

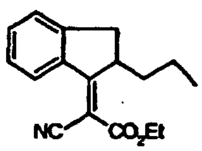

VII

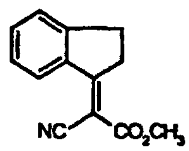

VIII

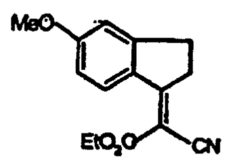

IX

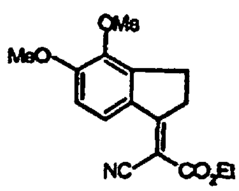

X

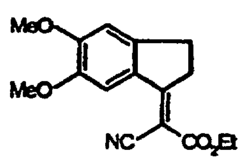

XI

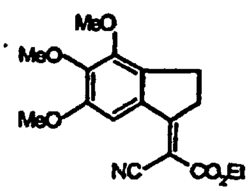

XII

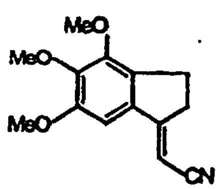

XIII

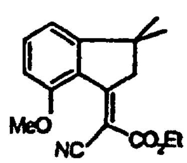

XIV

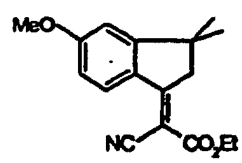

XV

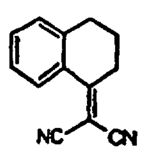

XVI

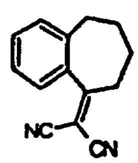

XVII

and having the general formula

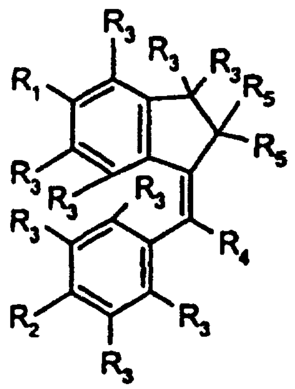

wherein

$R_1$, $R_2$ and $R_3$ can mutually independently be the same or different and denote hydrogen atom, hydroxy group or monovalent, optionally substituted organic radical and

$R_4$ denotes cyano group or monovalent, optionally substituted organic radical and

$R_5$ can be the same or different and denotes hydroxy group or monovalent, optionally substituted organic radical,

are excluded.

**13.** Compounds having the formula (Ib)

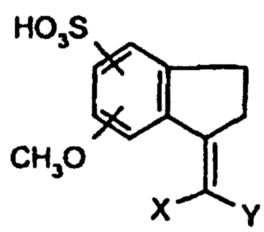

(Ib)

whereby

$R_5$ X and Y mutually independently denote CN, $CO_2R^9$, $CONR^9R^{10}$ or $COR^9$, whereby $R^9$ and $R^{10}$ mutually independently stand for hydrogen, $C_1$ to $C_8$ alkyl or $C_5$ to $C_{10}$cycloalkyl and furthermore one of the radicals,

X or Y can additionally be a $C_1$ to $C_8$ alkyl radical, a $C_5$ to $C_{10}$ aryl radical, in particular phenyl, or a 5- to 6-membered heteroaryl radical containing 1 to 2 heteroatoms from the series N, O, S, or

X and Y together with the β-atom to which they are bonded denote a 5- to 7-membered ring containing up to 3 heteroatoms, in particular oxygen and/or nitrogen, whereby the ring atoms can be substituted, particularly with exocyclically double-bonded oxygen (keto group) preferably in a vicinal position to the β-atom.

## Revendications

**1.** Préparations cosmétiques et pharmaceutiques contenant comme absorbants d'UV des composés d'indanylidène

de formule (I)

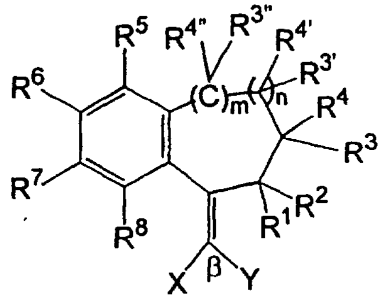

(I)

dans laquelle
$R^1$ à $R^4$, $R^{3'}$, $R^{3''}$, $R^{4'}$ et $R^{4''}$ signifient indépendamment l'hydrogène, un radical alkyle en $C_1$-$C_8$ ou cycloalkyle en $C_5$-$C_{10}$, sous réserve que deux substituants sur des atomes de C adjacents ensemble peuvent également signifier un groupe alkylène en $C_1$-$C_4$ éventuellement substitué, en particulier un groupe alkylène en $C_3$-$C_4$, où un groupe méthylène peut être remplacé par -O-, -S-, ou -NH-, et en outre indépendamment l'un de l'autre signifient un groupe alcoxy en $C_1$-$C_4$, hydroxy, carboxy, carbalcoxy ou carbamoyle,
$R^5$ à $R^8$ indépendamment l'un de l'autre ont la signification de $R^1$, $R^2$ ou sulfo ou aminosulfonyle,
X et Y indépendamment l'un de l'autre signifient CN, $CO_2R^9$, $CONR^9R^{10}$ ou $COR^9$, où $R^9$ et $R^{10}$ indépendamment l'un de l'autre signifient l'hydrogène, un radical alkyle en $C_1$ à $C_8$ ou cycloalkyle en $C_5$-$C_{10}$ et en outre un des radicaux,
X ou Y signifie en outre un reste alkyle en $C_1$-$C_8$, un reste aryle en $C_5$-$C_{10}$, en particulier phényle, ou un reste hétéroaryle de 5 à 6 chaînons qui peut contenir 1 à 2 hétéroatomes de la série de N, O, S, ou
X et Y ensemble avec l'atome bêta auquel ils sont liés, signifient un cycle de 5 à 7 chaînons, qui contient jusqu'à 3 hétéroatomes, en particulier l'oxygène et/ou l'azote, les atomes du cycle pouvant être substitués, en particulier avec l'oxygène lié par double liaison en dehors du cycle (groupe céto), de préférence en position adjacente de l'atome bêta, et
n, m indépendamment l'un de l'autre valent zéro ou 1.

2. Préparations cosmétiques et pharmaceutiques selon la revendication 1 contenant comme absorbants d'UV des composés d'indanylidène de formule (I)

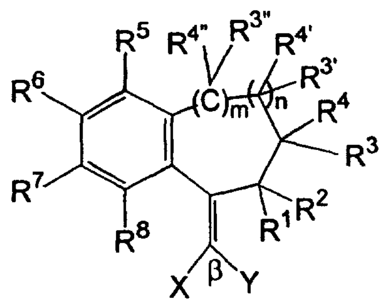

(I)

dans laquelle
$R^1$ à $R^4$, $R^{3'}$, $R^{3''}$, $R^{4'}$ et $R^{4''}$ signifient indépendamment l'hydrogène, un radical alkyle en $C_1$-$C_8$ ou cycloalkyle en $C_5$-$C_{10}$, sous réserve que deux substituants sur des atomes de C adjacents ensemble peuvent également signifier un groupe alkylène en $C_1$-$C_4$ éventuellement substitué, en particulier un groupe alkylène en $C_3$-$C_4$, où un groupe méthylène peut être remplacé par -O-, -S-, ou -NH-, et en outre indépendamment l'un de l'autre signifient un groupe alcoxy en $C_1$-C4, hydroxy, carboxy, carbalcoxy ou carbamoyle,
$R^5$ à $R^8$ indépendamment l'un de l'autre ont la signification de $R^1$, $R^2$ ou sulfo ou aminosulfonyle,
X signifie un groupe cyano et
Y signifie un groupe carbalcoxy en $C_1$-$C_4$.

**3.** Préparations cosmétiques et pharmaceutiques selon les revendications 1 et 2 contenant comme absorbants d'UV des composés d'indanylidène de formules

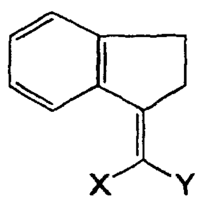

(Ia)

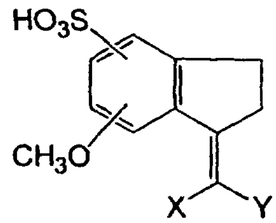

(Ib)

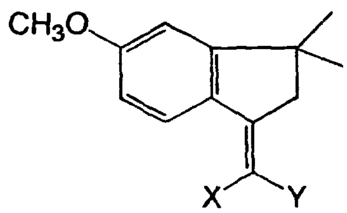

(Ic)

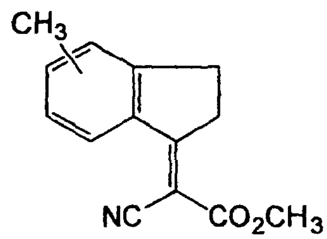

(Id)

dans lesquelles
X est un groupe cyano et
Y est un groupe carbalcoxy en $C_1$-$C_4$.

**4.** Préparations cosmétiques et pharmaceutiques selon les revendications 1 à 3 contenant 0,5 à 15% en poids des composés d'indanylidène rapporté au poids total de la préparation.

**5.** Préparations cosmétiques et pharmaceutiques selon les revendications 1 à 4 contenant 1 à 5% en poids de 4-tert.-butyl-4-méthoxydibenzoyl-méthane et au moins 1% en poids du composé d'indanylidène rapporté au poids total de la préparation.

**6.** Préparations cosmétiques et pharmaceutiques selon les revendications 1 à 4 contenant 1 à 10% en poids d'ester éthylhexylique de l'acide p-méthoxycinnamique ou d'ester isoamylique de l'acide p-méthoxycinnamique et au moins 1% en poids du composé d'indanylidène rapporté au poids total de la préparation.

**7.** Utilisation de préparations cosmétiques et pharmaceutiques selon les revendications 4 à 6 pour la protection de la peau et des cheveux du rayonnement UV.

**8.** Utilisation comme absorbants d'UV de composés d'indanylidène de formule (I)

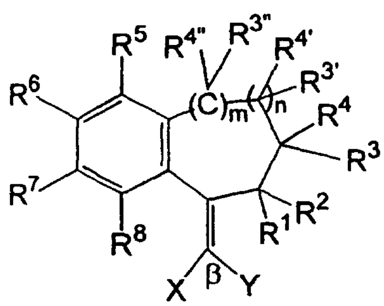

(I)

dans laquelle
$R^1$ à $R^4$, $R^{3'}$, $R^{3''}$, $R^{4'}$ et $R^{4''}$ signifient indépendamment l'hydrogène, un radical alkyle en $C_1$-$C_8$ ou cycloalkyle en $C_5$-$C_{10}$, sous réserve que deux substituants sur des atomes de C adjacents ensemble peuvent également signifier un groupe alkylène en $C_1$-$C_4$ éventuellement substitué, en particulier un groupe alkylène en $C_3$-$C_4$, où un groupe méthylène peut être remplacé par -O-, -S-, ou -NH-, et en outre indépendamment l'un de l'autre signifient un groupe alcoxy en $C_1$-$C_4$, hydroxy, carboxy, carbalcoxy ou carbamoyle,
$R^5$ à $R^8$ indépendamment l'un de l'autre ont la signification de $R^1$, $R^2$ ou sulfo ou aminosulfonyle,
X et Y indépendamment l'un de l'autre signifient CN, $CO_2R^9$, $CONR^9R^{10}$ ou $COR^9$, où $R^9$ et $R^{10}$ indépendamment l'un de l'autre signifient l'hydrogène, un radical alkyle en $C_1$ à $C_8$ ou cycloalkyle en $C_5$-$C_{10}$ et en outre un des radicaux,
X ou Y signifie en outre un reste alkyle en $C_1$-$C_8$, un reste aryle en $C_5$-$C_{10}$, en particulier phényle, ou un reste hétéroaryle à 5 à 6 chaînons qui peut contenir 1 à 2 hétéroatomes de la série de N, O, S, ou
X et Y ensemble avec l'atome bêta auquel ils sont liés, signifient un cycle de 5 à 7 chaînons, qui contient jusqu'à 3 hétéroatomes, en particulier l'oxygène et/ou l'azote, les atomes du cycle pouvant être substitués, en particulier avec l'oxygène lié par double liaison en dehors du cycle (groupe céto), de préférence en position adjacente de l'atome bêta, et
n, m indépendamment l'un de l'autre valent zéro ou 1.

**9.** Utilisation selon la revendication 8 comme absorbants d'UV dans des préparations cosmétiques ou pharmaceutiques.

**10.** Utilisation selon les revendications 8 et 9 comme absorbants d'UV dans des agents de protection solaire.

**11.** Utilisation selon les revendications 8 à 10 comme absorbants d'UV dans une combinaison avec des pigments finement divisés dans des produits de protection solaire et de soins journaliers.

**12.** Composés indanylidènes de formule (I)

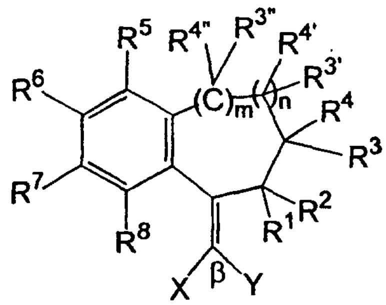

(I)

dans laquelle
$R^1$ à $R^4$, $R^{3'}$, $R^{3''}$, $R^{4'}$ et $R^{4''}$ signifient indépendamment l'hydrogène, un radical alkyle en $C_1$-$C_8$ ou cycloalkyle en $C_5$-$C_{10}$, sous réserve que deux substituants sur des atomes de C adjacents ensemble peuvent également signifier un groupe alkylène en $C_1$-$C_4$ éventuellement substitué, en particulier un groupe alkylène en $C_3$-$C_4$, où un groupe méthylène peut être remplacé par -O-, -S-, ou -NH-, et en outre indépendamment l'un de l'autre signifient un groupe alcoxy en $C_1$-$C_4$, hydroxy, carboxy, carbalcoxy ou carbamoyle,
$R^5$ à $R^8$ indépendamment l'un de l'autre ont la signification de $R^1$, $R^2$ ou sulfo ou aminosulfonyle,
X et Y indépendamment l'un de l'autre signifient CN, $CO_2R^9$, $CONR^9R^{10}$ ou $COR^9$, où $R^9$ et $R^{10}$ indépendamment l'un de l'autre signifient l'hydrogène, un radical alkyle en $C_1$ à $C_8$ ou cycloalkyle en $C_5$-$C_{10}$ et en outre un des radicaux,
X ou Y en outre signifie un reste alkyle en $C_1$-$C_8$, un reste aryle en $C_5$-$C_{10}$, en particulier phényle, ou un reste hétéroaryle de 5 à 6 chaînons qui peut contenir 1 à 2 hétéroatomes de la série de N, O, S, ou
X et Y ensemble avec l'atome bêta auquel ils sont liés, signifient un cycle de 5 à 7 chaînons, qui contient jusqu'à 3 hétéroatomes, en particulier l'oxygène et/ou l'azote, les atomes du cycle pouvant être substitués, en particulier avec l'oxygène lié par double liaison en dehors du cycle (groupe céto), de préférence en position adjacente de l'atome bêta, et
n, m indépendamment l'un de l'autre valent zéro ou 1, les composés ayant les formules

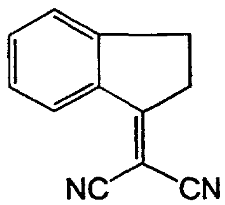

I

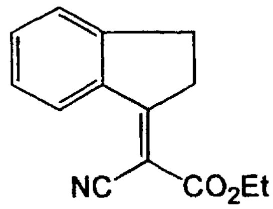

II

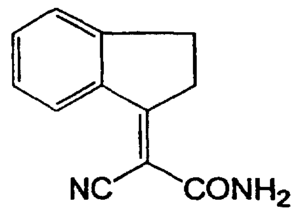

III

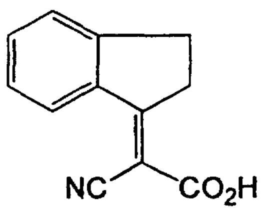

IV

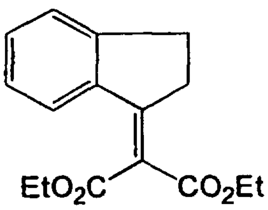

V

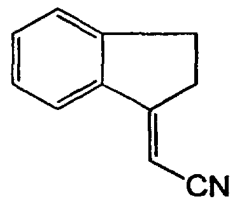

VI

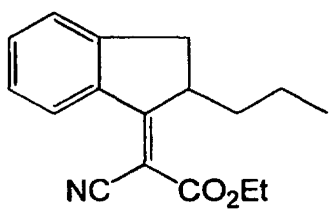

VII

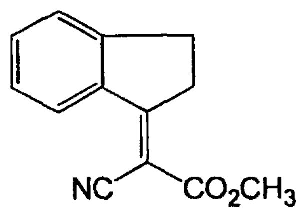

VIII

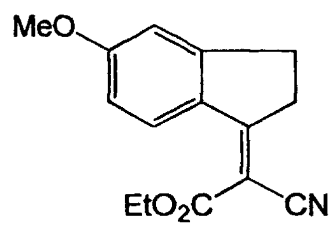

IX

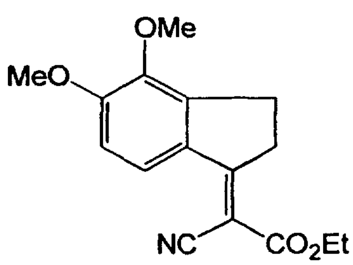

X

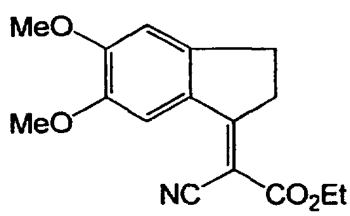

XI

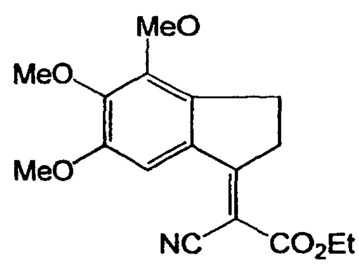

XII

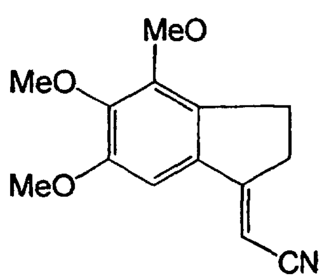

XIII

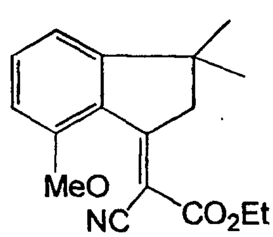

XIV

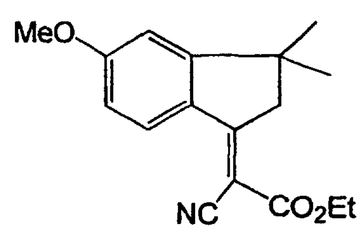

XV

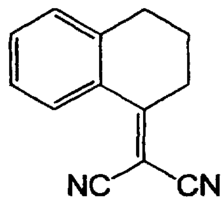

XVI

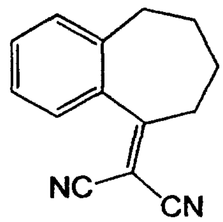

XII

ainsi que ceux de formule

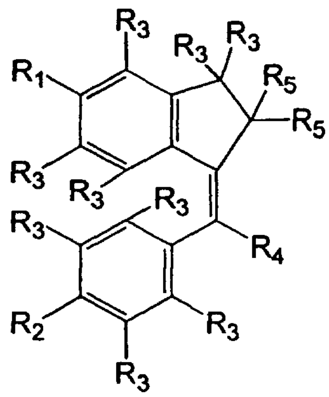

dans laquelle

$R_1$, $R_2$ et $R_3$ chaque fois indépendamment l'un de l'autre peuvent être identiques ou différents et sont un atome d'hydrogène, un groupe hydroxy ou un reste organique monovalent, éventuellement substitué, et

$R_4$ est un groupe cyano ou un reste organique monovalent, éventuellement substitué et

$R_5$ peut être identique ou différente et est un groupe hydroxy ou un reste organique monovalent, éventuellement substitué, étant exclus.

**13.** Composés de formule (Ib)

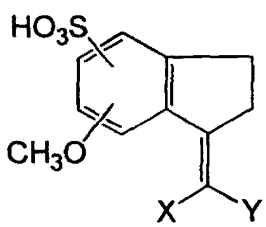

**(Ib)**

dans laquelle

X et Y indépendamment l'un de l'autre signifient CN, $CO_2R^9$, $CONR^9R^{10}$ ou $COR^9$, où $R^9$ et $R^{10}$ indépendamment l'un de l'autre signifient l'hydrogène, un radical alkyle en $C_1$ à $C_8$ ou cycloalkyle en $C_5$-$C_{10}$ et en outre un des radicaux,

X ou Y en outre un reste alkyle en $C_1$-$C_8$, un reste aryle en $C_5$-$C_{10}$, en particulier phényle, ou un reste hétéroaryle de 5 à 6 chaînons qui peut contenir 1 à 2 hétéroatomes de la série de N, O, S, ou

X et Y ensemble avec l'atome bêta auquel ils sont liés, signifient un cycle de 5 à 7 chaînons, qui contient jusqu'à 3 hétéroatomes, en particulier l'oxygène et/ou l'azote, les atomes du cycle pouvant être substitués, en particulier avec l'oxygène lié par double liaison en dehors du cycle (groupe céto), de préférence en position adjacente de l'atome bêta.